# EUROPEAN PATENT APPLICATION

(11) **EP 2 072 487 A1**
(43) Date of publication of application: **24.06.2009**
(21) Application number: 07254885.2
(22) Date of filing: 17.12.2007
(51) Int. Cl.: C07C 29/151, C07C 31/08

(54) **Process for the conversion of hydrocarbons to ethanol**

(71) Applicant: BP p.l.c., London SW1Y 4PD (GB)
(72) Inventor: The designation of the inventor has not yet been filed
(74) Representative: De Kezel, Eric

(57) **Abstract**

The present invention relates to a process for the conversion of a carbonaceous feedstock to ethanol.

## Description

The present invention relates to a process for the conversion of a carbonaceous feedstock to ethanol.

In recent years increased use and demand for alcohols such as methanol, ethanol and higher alcohols has led to a greater interest in processes relating to alcohol production. The said alcohols may be produced by the fermentation of, for example, sugars and/or cellulosic materials.

Alternatively alcohols, such as ethanol, may be produced from synthesis gas. Synthesis gas refers to a combination of hydrogen and carbon oxides produced in a synthesis gas plant from a carbon source such as natural gas, petroleum liquids, biomass and carbonaceous materials including coal, recycled plastics, municipal wastes, or any organic material. Thus, alcohol and alcohol derivatives may provide non-petroleum based routes for the production of valuable chemicals and fuels.

Generally, the production of alcohols, for example methanol, takes place via three process steps: synthesis gas preparation, methanol synthesis, and methanol purification. In the synthesis gas preparation step, an additional stage maybe employed whereby the feedstock is treated, e.g. the feedstock is purified to remove sulphur and other potential catalyst poisons prior to being converted into synthesis gas. This treatment can also be conducted after syngas preparation; for example, when coal or biomass is employed.

The reaction to produce alcohol(s) from syngas is generally exothermic. The formation of C2 and C2+ alcohols is believed to proceed via the formation of methanol for modified methanol catalysts and cobalt molybdenum sulphide catalysts. However, the production of methanol is equilibrium limited and thus requires high pressures in order to achieve viable yields. Hence, pressure can be used to increase the yield, as the reaction which produces methanol exhibits a decrease in volume, as disclosed in U.S. Pat. No. 3,326,956. Improved catalysts have now allowed viable rates of methanol formation to be achieved at reduced reaction temperatures, and hence allow commercial operation at lower reaction pressures, e.g. a copper oxide-zinc oxide-alumina catalyst that typically operates at a nominal pressure of 5-10 MPa and temperatures ranging from approximately 150 DEG C. to 450 DEG C. over a variety of catalysts, including CuO/ZnO/Al2 03, CuO/ZnO/Cr2 03, ZnO/Cr2O3, and supported Fe, Co, Ni, Ru, Os, Pt, and Pd catalysts. A low-pressure, copper-based methanol synthesis catalyst is commercially available from suppliers such as BASF, ICI Ltd. of the United Kingdom, and Haldor-Topsoe. Methanol yields from copper-based catalysts are generally over 99.5% of the converted CO+CO2 present. Water is a by-product of the conversion of CO2 to methanol and the conversion of CO synthesis gas to C2 and C2+ oxygenates. In the presence of an active water gas-shift catalyst, such as a methanol catalyst or a cobalt molybdenum catalyst the water equilibrates with the carbon monoxide to give CO2 and hydrogen. A paper entitled, "Selection of Technology for Large Methanol Plants," by Helge Holm-Larsen, presented at the 1994 World Methanol Conference, Nov. 30-Dec. 1, 1994, in Geneva, Switzerland, reviews the developments in methanol production and shows how further reduction in costs of methanol production will result in the construction of very large plants with capacities approaching 10,000 metric tonnes per day.

Other processes, for the production of C2 and C2+ alcohol(s), include the processes described hereinafter; US 4,122,110 relates to a process for manufacturing alcohols, particularly linear saturated primary alcohols, by reacting carbon monoxide with hydrogen at a pressure between 20 and 250 bars and a temperature between 150 and 400 DEG C., in the presence of a catalyst, characterized in that the catalyst contains at least 4 essential elements: (a) copper (b) cobalt (c) at least one element M selected from chromium, iron, vanadium and manganese, and (d) at least one alkali metal.

US 4,831,060 relates to the production of mixed alcohols from carbon monoxide and hydrogen gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulfiding. The composition of the mixed alcohols fraction can be selected by selecting the extent of intimate contact among the catalytic components.

Journal of Catalysis 114, 90-99 (1988) discloses a mechanism of ethanol formation from synthesis gas over CuO/ZnO/Al2O3. The formation of ethanol from carbon monoxide and hydrogen over a CuO/ZnO methanol catalyst is studied in a fixed-bed microreactor by measuring the isotopic distribution of the carbon in the product ethanol when isotopically-enriched C¹³ methanol was added to the feed.

As the importance of ethanol is ever increasing in today's world, so is the need and desire to produce ethanol with a higher carbon efficiency, a higher conversion and an improved selectivity from a carbonaceous feedstock. Hence, the present invention provides a process that allows one to produce ethanol from a carbonaceous feedstock, with improved carbon efficiency, a higher selectivity and in particular, with a more efficient conversion to ethanol.

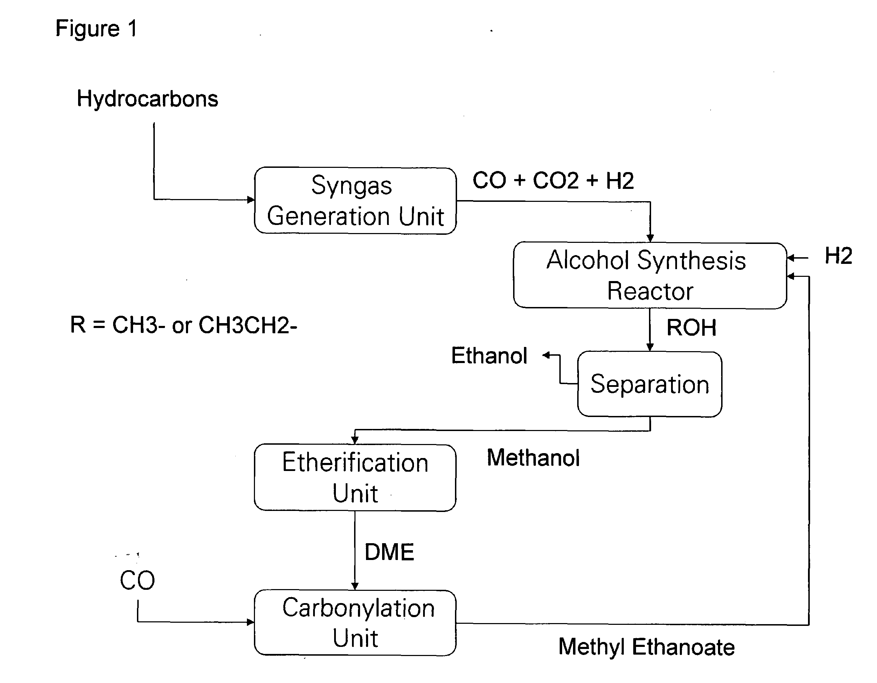

Figure 1 represents an embodiment of a process scheme according to the present invention, wherein the references correspond to those used in the present description and appending claims.

Thus, the present invention relates to a process for the conversion of synthesis gas into ethanol characterised by the following steps:
1) converting synthesis gas and methyl ethanoate in an alcohol synthesis unit to produce methanol and ethanol,
2) separating the methanol from the ethanol in step 1,
3) introducing methanol from step 2 into an etherification unit to produce dimethyl ether,
4) introducing dimethyl ether, from step 3 together with carbon monoxide, into a carbonylation unit, in the presence of a iridium based carbonylation catalyst, to produce methyl ethanoate,
5) introducing methyl ethanoate, from step 4, into the alcohol synthesis unit of step 1, and
6) recovering ethanol from step 2.

Furthermore, the present invention relates to a process for the conversion of a hydrocarbon feedstock(s) into ethanol, wherein the hydrocarbons are first converted into synthesis gas, which is subsequently converted into ethanol, characterised by the following steps:
1) introducing a hydrocarbon feedstock, into a synthesis gas reactor, in order to produce a mixture of carbon oxide(s) and hydrogen (synthesis gas),
2) converting synthesis gas, from step 1, together with hydrogen and methyl ethanoate in an alcohol synthesis unit to produce methanol and ethanol,
3) separating the methanol from the ethanol in step 2,
4) introducing methanol from step 3 into an etherification unit, to produce dimethyl ether,
5) introducing dimethyl ether, from step 4 together with carbon monoxide, into a carbonylation unit, in the presence of a iridium based carbonylation catalyst, to produce methyl ethanoate,
6) introducing methyl ethanoate, from step 5, into the alcohol synthesis unit of step 2, and
7) recovering ethanol from step 3.

According to one aspect of the present invention, the synthesis gas feedstock, a mixture of carbon oxide(s) and hydrogen, that is used to produce the methanol feed stream, is preferably produced from a hydrocarbon feedstock.

The hydrocarbon feedstock is preferably a carbonaceous material, for example biomass, plastic, naphtha, refinery bottoms, crude syngas (from underground coal gasification or biomass gasification), smelter off gas, municipal waste, coal bed methane, coal, and/or natural gas, with coal and natural gas being the preferred sources, and natural gas being the most preferable source. To one skilled in the art a combination of sources can also be used, for example coal and natural gas to advantageously increase the hydrogen to carbon ratio.

Natural gas commonly contains a range of hydrocarbons (e.g. C1-C3 alkanes), in which methane predominates. In addition to this, natural gas will usually contain nitrogen, carbon dioxide and sulphur compounds. Preferably the nitrogen content of the feedstock is less than 40 wt %, more preferably less than 10 wt % and most preferably less than 1 wt %.

Processes for producing mixtures of carbon oxide(s) and hydrogen (commonly known as synthesis gas), in a syngas reactor, are well known. Each method has its advantages and disadvantages, and the choice of using a particular reforming process over another is governed by economic and available feed stream considerations, as well as by the desire to obtain the optimum (H2-CO2):(CO+CO2) molar ratio in the resulting synthesis gas that is suitable for further chemical processing. A discussion of the available synthesis gas production technologies is provided in both "Hydrocarbon Processing" V78, N.4, 87-90, 92-93 (April 1999) and "Petrole et Techniques", N. 415, 86-93 (July-August 1998), and are both hereby incorporated by reference.

.It is also known that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

Typically, for commercial syngas production the pressure at which the synthesis gas is produced from a steam reformer ranges from approximately 0.1 to 10 MPa, preferably 2 to 3 MPa and the temperatures at which the synthesis gas exits the reformer ranges from approximately 700 to 1000 DEG C. Likewise, for commercial syngas production the pressure at which the synthesis gas is produced from an auto-thermal reformer ranges from approximately 0.1 to 10 MPa, preferably 2 to 5 MPa and the temperatures at which the synthesis gas exits the reformer ranges from approximately 700 to 1300 DEG C. Where the high temperatures are necessary in order to produce a favourable equilibrium for syngas production, and to avoid metallurgy problems associated with carbon dusting. The synthesis gas contains a molar ratio of (H2-CO2):(CO+CO2) ranging from 0.8 to 3.0, which is dependent on the hydrocarbon feedstock(s) and the method of reforming used. For example, when natural gas is used as the hydrocarbon feedstock for steam reforming, the syngas obtained usually has a maximum (H2-CO2):(CO+CO2) ratio of 3.0. However, when natural gas is used as the hydrocarbon feedstock for auto-thermal reforming, the syngas obtained usually has a (H2-CO2):(CO+CO2) ratio of 1.5.

According to a preferred embodiment of the present invention, when ethanol (and optionally methanol) are the targeted products, the molar ratio, (H2-CO2):(CO+CO2), of the syngas stream exiting the syngas generation unit(s) is greater than 1.6, more preferably greater than 1.8 and most preferably greater than 2.0. Preferably, the molar ratio, (H2-CO2):(CO+CO2), of said syngas stream exiting the syngas generation unit(s) is less than 3.0, preferably less than 2.75, more preferably less than 2.4 and most preferably less than 2.2.

Furthermore, since the present invention allows to target multiple products, such as ethanoic acid and/or ethyl ethanoate and/or methyl ethanoate, together with said ethanol (and optionally methanol), the (H2-CO2):(CO+CO2) of the synthesis gas required to synthesise the said acid and ester products is preferably lower than the aforementioned values.

According to another embodiment of this invention when the hydrocarbon feed stock used for syngas generation is not a aliphatic hydrocarbon (e.g. coal, aromatic material, biomass e.g. fermentation residues) the molar ratio (H2-CO2):(CO+CO2) of the exit syngas is preferably adjusted to the target value by addition of extra hydrogen and/or removal of CO2.

CO2 may be removed by the use of a simple, yet effective, separation method known to those skilled in the art, for example, a "membrane separation method". Such membrane technologies can be found in 'Purification and Recovery Options for Gasification' D. J. Kubek, E. Polla, F. P. Wilcher, UOP, 1996, and are hereby incorporated by reference.

Alternatively, CO2 may be recovered and removed by any suitable method(s) known to those skilled in the art, for example, by reacting with amines; performing a methanol wash (i.e. the RECTISOL process) and/or by using hot potassium carbonate (e.g. the BENFIELD process).

According to a preferred embodiment of the present invention, the exit stream obtained from the syngas reactor (e.g. using a steam reformer), comprises essentially a mixture of carbon oxide(s) and hydrogen. It can also comprise water, nitrogen and traces of unconverted hydrocarbons (e.g. C1-C3 alkanes).

According to a preferred embodiment of the present invention, during synthesis gas generation, an additional stage may be employed whereby the feedstock is first purified to remove sulphur and other potential catalyst poisons (such as halides or metals e.g. Hg) prior to being converted into synthesis gas; alternatively this treatment can also be performed after syngas preparation for example, when coal or biomass are used.

According to the present invention, at least part of the said synthesis gas stream is then introduced into an alcohol synthesis unit, together with methyl and optionally ethyl ethanoate (known herewith and hereinafter as the ethanoates), to produce a stream comprising methanol and ethanol, in addition to unreacted methyl ethanoate and ethyl ethanoate.

Additionally by-products such as methane and higher alcohols may also be produced during alcohol synthesis (i.e. methanol and ethanol synthesis). According to a preferred embodiment of this aspect of the present invention, the stream exiting the alcohol synthesis unit is subsequently purified to remove said by-products by any methods known to those in the art to obtain substantially pure methanol and ethanol products.

For the targeted production of ethanol the preferred molar ratio, ([H2]-[CO2])/([CO]+[CO2]), (where the concentrations of relevant components are expressed in volume percent or mole percent), of the fresh synthesis gas feed stream fed into the alcohol synthesis unit is greater than 3.8, more preferably greater than 4.0 and most preferably greater than 4.1. Preferably the molar ratio, ([H2]-[CO2])/([CO]+[CO2]), of the fresh synthesis gas feed stream fed into the alcohol synthesis unit is less than 6.0, preferably less than 5.0 and most preferably less than 4.4.

To obtain the above high synthesis gas molar ratios, additional hydrogen needs to be added to the synthesis gas feed stream exiting the synthesis gas generation unit. Preferably, the said hydrogen is obtained from the aforementioned syngas generation stage. This is preferably performed by first removing carbon dioxide and water from the generated synthesis gas followed by a cryogenic separation to isolate the substantially pure carbon monoxide from the hydrogen. Alternative methods of separation, such as membrane separation technologies can also be employed. Alternatively, said hydrogen stream may also be obtained from another suitable source, such as another chemical process (e.g. off-gas from steel manufacture or electrolysis). Said hydrogen stream(s) may still contain inert impurities such as methane, nitrogen, noble gases, water and C 1 to C4 paraffinic hydrocarbons, which are preferably removed before use.

An added advantage of the present invention, when compared to other processes in the field, is that the purification of the separated hydrogen feed produced during synthesis gas generation has no requirement for the removal of carbon oxides. Indeed, this is advantageous when compared to a process with a separate hydrogenation stage for ethanoates where the carbon oxides act as poisons to the hydrogenation catalyst as well as increasing the formation of inert materials, thus necessitating an increased purge step.

According to a preferred embodiment of the present invention, the alcohol synthesis unit may be any reactor that is suitable for producing methanol and ethanol, for example a fluidised bed reactor or a fixed bed reactor, which can be run with or without external heat exchange equipments e.g. a multi-tubular reactor; or a fluidised bed reactor; or a void reactor.

Preferably the alcohol synthesis unit is operated at a temperature of more than 180°C, preferably more than 200°C and most preferably more than 220°C; and less than 290°C, preferably less than 280 °C and most preferably less than 270°C. Preferably the alcohol synthesis unit is operated at pressure of more than 2 MPa and preferably more than 5 MPa; and less than 10 MPa and preferably less than 9MPa. In fact, since methanol synthesis is an exothermic reaction, the chosen temperature of operation is governed by a balance of promoting the forward reaction (i.e. by not adversely affecting the equilibrium) and aiding the rate of conversion (i.e. higher productivity). The ethanol synthesis is also exothermic; however, in this case the chosen temperature of operation is governed by a balance of reaction rate and selectivity.

The preferred catalyst for the alcohol synthesis can be chosen amongst traditional methanol synthesis catalyst selected from one of the two following groups:
i. the high pressure zinc catalysts, composed of zinc oxide and a promoter; and
ii. low pressure copper catalysts, composed of zinc oxide, copper oxide , and a promoter.

A preferred methanol synthesis catalyst is a mixture of copper, zinc oxide, and a promoter such as, chromia or alumina. The Applicants have unexpectedly found that the said methanol synthesis catalyst demonstrated high hydrogenation activities.

According to a preferred embodiment of the present invention, the preferred catalyst consists of a mixture of the above methanol catalyst together with a hydrogenation catalyst selected from
(i) a precious metal based catalyst, comprising of at least one noble metal from Group VIII of the periodic table (CAS version, for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII noble metal;
(ii) a copper-based catalyst (for example a copper chromite or a mixed copper metal oxide based catalyst wherein the second metal can be Cu, Zn, Zr or Mn), and
(iii) mixtures thereof.

According to a preferred embodiment of the present invention, the hydrogenation catalyst mixed with the methanol catalyst is a copper based catalyst. Where, the preferred copper based catalyst is a supported catalyst which comprises copper, and preferably promoters, such as cobalt and/or manganese and/or chromium.

All of the aforementioned hydrogenation catalysts may advantageously be supported on any suitable support known to those skilled in the art; non-limiting examples of such supports include carbon, silica, titania, clays, aluminas, zinc oxide, zirconia and mixed oxides. Preferably, the palladium based catalyst is supported on carbon. Preferably, the copper based catalyst is supported on zinc oxide.

A key feature of the present invention is that both synthesis of methanol and hydrogenation of ethanoates into its corresponding alcohols occur in the same reactor. For the avoidance of doubt, when it is hereinabove referred to as a mixture of a methanol catalyst and a hydrogenation catalyst, it also covers physical blends of the two catalysts and/or separate packed zones of the two catalysts in the same reactor(s); according to a preferred mode of operation, the methanol synthesis catalyst is located at the inlet of the reactor while the hydrogenation catalyst is located downstream of said methanol synthesis catalyst.

According to the present invention, at least a part of the stream exiting the alcohol synthesis unit is passed through a separation unit (e.g. a separation column) to recover and collect a stream comprising the targeted ethanol and a stream comprising methanol.

This separation stage may be performed by any suitable means known to those skilled in the art, e.g. a sieve tray column, a packed column, a bubble cap column or a combination thereof.

Since methyl ethanoate and ethyl ethanoate can also be present in the exit stream from the alcohol synthesis unit, the Applicants have found the following preferred mode of operation(s):
(i) methanol/methyl ethanoate close boiling point mixture can be easily recovered together with the methanol and fed directly into the downstream etherification unit, and/or
(ii) ethanol/ethyl ethanoate azeotrope can be advantageously recovered and recycled to the alcohol synthesis unit.

Additionally by-products such as methane, ethane and other higher alcohols may also be produced during alcohol synthesis. According to a preferred embodiment of this aspect of the present invention, the streams exiting the separation zone are subsequently purified to remove said by-products from the methanol and ethanol streams by any methods known to those in the art.

According to the present invention, methanol is then introduced into an etherification unit to produce dimethyl ether. For example, dimethyl ether is commonly produced by the Lewis or Bronsted acid catalysed etherification of methanol; numerous catalysts for this reaction have been reported e.g Houben-Weyl, vol. VI/3, part 3, Georg Thieme Verlag, Stuttgart 1965, pp. 17, 18. Dr. Alexander Wacker, Gesellschaft für elektrochemische Industrie GmbH, DE 680 328, 1934 (P. Halbig, O. Moldenhauer). R. L. Brown, W. W. Odells, US 1 873 537, 1927. N. V. de Bataafsche Petroleum Maatschappij, FR 701 335, 1930; GB 332 756, 1929;GB 350 010, 1931;GB 403 402, 1932. Further examples of etherification catalysts include iron chloride, copper sulphate, copper chloride, manganese chloride, aluminium chloride, aluminium sulphate, chromium sulphate, alums, thorium compounds, aluminium oxide, titanium oxide, barium oxide, silica gel, and aluminium phosphate, acidic ionic liquids.

According to the present invention the preferred etherification catalysts are heterogeneous catalysts, such as aluminium oxides and aluminium silicate, which can be modified by doping. Corresponding etherification catalysts and process operating conditions that may be advantageously used according to the present invention are described in Mobil Oil Corporation, DE 2 818 831, 1978 (F. G. Dwyer, A. B. Schwartz); DE-OS 3 201 155, 1982 (W. K. Bell, C. Chang); Du Pont, EP-A 99 676,1983 (D. L. Brake); Mitsubishi Chemical Industries, EP-A 124 078, 1984 (N. Murai, K. Nakamichi, M. Otake, T. Ushikubo).

Zeolites, strong acid ion exchange resins and supported heteropolyacids (such as silicotungstenic acid) can also be advantageously used as etherification catalysts, supported heteropolyacids catalysts being the preferred one used according to the present invention..

During the methanol etherification process to dimethyl ether, water is also produced. It is preferred according to the present invention to proceed with the removal of said water prior to introduction the dimethyl ether to the next stage of this invention. However, some water may advantageously be introduced together with the dimethyl ether into the carbonylation unit as explained hereafter.

According to the present invention, at least a part of the aforementioned stream comprising dimethyl ether (DME) together with a substantially pure carbon monoxide stream, are introduced into a carbonylation reactor in the presence of an iridium based catalyst. Preferably at least part, most preferably all, of the said DME stream, emanates from the aforementioned etherification unit.

Preferably at least a part of the said carbon monoxide stream is obtained from the aforementioned synthesis gas generation stage. This is preferably performed by first removing carbon dioxide and water from the generated synthesis gas followed by a cryogenic separation to isolate the substantially pure carbon monoxide from the hydrogen. A particular advantage according to the present invention is that both the hydrogen fed to the alcohol synthesis unit and the carbon monoxide fed to the carbonylation unit, in the presence of an iridium base catalyst, are obtained from the same synthesis gas separation stage. Alternative methods of separation, such as membrane separation technologies can also be employed. Alternatively, said carbon monoxide stream may also be obtained from another suitable source, such as another chemical process (e.g. off-gas from steel manufacture).

Said substantially pure carbon monoxide stream may contain inert impurities such as carbon dioxide, methane, nitrogen, noble gases, water and C 1 to C4 paraffinic hydrocarbons which are preferably removed before use. The presence of hydrogen in the carbon monoxide and generated in situ by the water gas shift reaction is preferably kept low, for example, less than 0.1 MPa partial pressure, as its presence may result in the formation of hydrogenation products.

According to this aspect of the present invention, the step of introducing DME together with carbon monoxide, into a carbonylation reactor in the presence of an iridium based catalyst is performed under conditions favourable for producing methyl ethanoate.

There are many examples in the prior art which disclose carbonylation processes in the presence of iridium catalysts that can be suitably used in the present invention.

For example, such carbonylation iridium catalysts are described in US 3772380. UK patent GB 1276326 also describes the carbonylation of alcohols in the presence of iridium catalysts, halogen promoters and water or an alcohol, ether or ester.

Additionally carbonylation processes may be operated the presence of ruthenium and osmium catalysts together with iridium and may also be used in the present invention. Thus, UK patents GB 1234641 and GB 1234642 describe a process for the carbonylation of an alcohol in the presence of a promoted iridium catalyst. According to a preferred embodiment of this aspect of the present invention, the carbonylation process takes place in the presence of an iridium catalyst together with at least one promoter; indeed, such catalyst systems have proven to have beneficial effects on the rate of carbonylation of DME. Said carbonylation process is thus preferably performed in the presence of at least a finite concentration of water with a catalyst system comprising:

### (a) an iridium catalyst, (b) methyl iodide and (c) at least one promoter.

Thus, according to a preferred embodiment of this aspect of the present invention the process for the production of methyl ethanoate by carbonylation of DME comprises contacting DME with carbon monoxide, in the liquid reaction composition, in a carbonylation reactor wherein, the liquid reaction composition comprises:

### (a) ethanoic acid, (b) methyl ethanoate, (c) an iridium catalyst, (d) methyl iodide, (e) water and (f) at least one promoter.

According to an embodiment of this aspect of the present invention, during the carbonylation process water may be produced via by-product formation and/or generated during methane production. Water may be separated from other components of reaction composition withdrawn from the reactor; this separated water and/or additional water may be recycled in controlled amounts to maintain a preferred concentration of water in the liquid reaction composition. Preferably, the concentration of water in the liquid reaction composition of the carbonylation reactor is in the range 0.1 to 15% by weight, more preferably 1 to 10% by weight, most preferably 1 to 6.5% by weight.

The iridium catalyst in the liquid reaction composition may comprise any iridium containing compound which is soluble in the liquid reaction composition. The iridium catalyst may be added to the liquid reaction composition for the carbonylation reaction in any suitable form which dissolves in the liquid reaction composition or is convertible to a soluble form. Examples of suitable iridium-containing compounds which may be added to the liquid reaction composition include IrCl3, IrI3, IrBr3, [Ir(CO)2I]2, [Ir(CO)2Cl]2, [Ir(CO)2Br]2, [Ir(CO)2I2]⁻ H⁺, [Ir(CO)2Br2]⁻ H⁺, [Ir(CO)2I4]⁻ H⁺, [Ir(CH3)I3(CO)2]⁻ H⁺, Ir4(CO)12, IrCl3.3H2O, IrBr3.3H2O, Ir4(CO)12, iridium metal, Ir2O3, IrO2, Ir(acac)(CO)2, Ir(acac)3, iridium acetate, [Ir3O(OAc)6(H2O)3][OAc], and hexachloroiridic acid [H2IrC16], preferably, chloride-free complexes of iridium such as ethanoates, oxalates and acetoacetates which are soluble in one or more of the carbonylation reaction components such as water, alcohol and/or carboxylic acid. Particularly preferred is green iridium ethanoate which may be used in an ethanoic acid or aqueous ethanoic acid solution.

Preferably, the iridium carbonylation catalyst concentration in the liquid reaction composition is in the range 100 to 6000 ppm by weight of iridium, more preferably 700 to 3000 ppm by weight of iridium.

In the process of the present invention at least one promoter is present in the reaction composition. Suitable promoters are preferably selected from the group consisting of ruthenium, osmium, rhenium, cadmium, mercury, zinc, gallium, indium and tungsten, and are more preferably selected from ruthenium and osmium and most preferably is ruthenium. Preferably, the promoter is present in an effective amount up to the limit of its solubility in the liquid reaction composition and/or any liquid process streams recycled to the carbonylation reactor from the acetic acid recovery stage. The promoter is suitably present in the liquid reaction composition at a molar ratio of promoter:iridium of between 0.5:1 and 15:1. As noted above, the beneficial effect of a promoter such as ruthenium has been found to be greatest at the water concentration which gives the maximum carbonylation rate at any defined methyl ethanoate and methyl iodide concentration. A suitable promoter concentration is 400 to 5000 ppm.

The promoter may comprise any suitable promoter metal-containing compound which is soluble in the liquid reaction composition. The promoter may be added to the liquid reaction composition for the carbonylation reaction in any suitable form which dissolves in the liquid reaction composition or is convertible to soluble form.

Examples of suitable ruthenium-containing compounds which may be used as sources of promoter include ruthenium (III) chloride, ruthenium (III) chloride trihydrate, ruthenium (IV) chloride, ruthenium (III) bromide, ruthenium metal, ruthenium oxides, ruthenium (III) formate, [Ru(CO)3I3]⁻ H⁺, [Ru(CO)2I2]n, [Ru(CO)4I2], [Ru(CO)3I2]2, tetra(aceto)chlororuthenium(II,III), ruthenium (III) acetate, ruthenium (III) propionate, ruthenium (III) butyrate, ruthenium pentacarbonyl, trirutheniumdodecacarbonyl and mixed ruthenium halocarbonyls such as dichlorotricarbonylruthenium (II) dimer, dibromotricarbonylruthenium (II) dimer, and other organoruthenium complexes such as tetrachlorobis(4-cymene)diruthenium(II), tetrachlorobis(benzene)diruthenium(II), dichloro(cycloocta-1,5-diene)ruthenium (II) polymer and tris(acetylacetonate)ruthenium (III).

Examples of suitable osmium-containing compounds which may be used as sources of promoter include osmium (III) chloride hydrate and anhydrous, osmium metal, osmium tetraoxide, triosmiumdodecacarbonyl, [Os(CO)4I2], [Os(CO)3I2]2, [Os(CO)3I3]⁻ H⁺, pentachloro- mu -nitrodiosmium and mixed osmium halocarbonyls such as tricarbonyldichloroosmium (II) dimer and other organoosmium complexes.

Examples of suitable rhenium-containing compounds which may be used as sources of promoter include Re2(CO)10, Re(CO)5Cl, Re(CO)5Br, Re(CO)5I, ReCl3.xH2O, [Re(CO)4I]2, [Re(CO)4I2]⁻ H⁺, and ReCl5.yH2O.

Examples of suitable cadmium-containing compounds which may be used include Cd(OAc)2, CdI2, CdBr2, CdCl2, Cd(OH)2, and cadmium acetylacetonate.

Examples of suitable mercury-containing compounds which may be used as sources of promoter include Hg(OAc)2, HgI2, HgBr2, HgCl2, Hg2I2, and Hg2Cl2.

Examples of suitable zinc-containing compounds which may be used as sources of promoter include Zn(OAc)2, Zn(OH)2, ZnI2, ZnBr2, ZnCl2, and zinc acetylacetonate.

Examples of suitable gallium-containing compounds which may be used as sources of promoter include gallium acetylacetonate, gallium acetate, GaCl3, GaBr3, GaI3, Ga2Cl4 and Ga(OH)3.

Examples of suitable indium-containing compounds which may be used as sources of promoter include indium acetylacetonate, indium acetate, InCl3, InBr3, InI3, InI and In(OH)3.

Examples of suitable tungsten-containing compounds which may be used as sources of promoter include W(CO)6, WCl4, WCl6, WBr5, WI2, or C9H12 W(CO)3 and any tungsten chloro-,bromo- or iodo-carbonyl compound.

Preferably, the iridium- and promoter-containing compounds are free of impurities which provide or generate in situ ionic iodides which may inhibit the reaction, for example, alkali or alkaline earth metal or other metal salts.

Ionic contaminants such as, for example, (a) corrosion metals, particularly nickel, iron and chromium and (b) phosphines or nitrogen containing compounds or ligands which may quaternise in situ; should be kept to a minimum in the liquid reaction composition as these will have an adverse effect on the reaction by generating I⁻ in the liquid reaction composition which has an adverse effect on the reaction rate. Some corrosion metal contaminants such as for example molybdenum have been found to be less susceptible to the generation of I⁻. Corrosion metals which have an adverse affect on the reaction rate may be minimised by using suitable corrosion resistant materials of construction. Similarly, contaminants such as alkali metal iodides, for example lithium iodide, should be kept to a minimum. Corrosion metal and other ionic impurities may be reduced by the use of a suitable ion exchange resin bed to treat the reaction composition, or preferably a catalyst recycle stream. Such a process is described in US 4007130. Preferably, ionic contaminants are kept below a concentration at which they would generate 500 ppm [I]⁻, preferably less than 250 ppm [I]⁻ in the liquid reaction composition.

Preferably, the concentration of methyl iodide in the liquid reaction composition is in the range 1 to 20% by weight, preferably 5 to 16% by weight.

The partial pressure of carbon monoxide in the carbonylation reactor is suitably in the range 0.1 to 7 MPa preferably 0.1 to 3.5 MPa and most preferably 0.1 to 1.5 MPa.

The catalyst system used in the carbonylation process of the present invention has been found to be particularly beneficial at relatively low partial pressures of carbon monoxide where the rate of reaction may be dependent upon the carbon monoxide partial pressure. Under these conditions, it has been found that the catalyst system has the advantage of providing an increased rate of reaction over catalyst systems without the promoters of the present invention. This advantage allows for an increased rate of reaction under conditions when the carbon monoxide partial pressure is relatively low, for example due to a low total pressure in the carbonylation reactor or due to high vapour pressure of components of the liquid reaction composition, e.g. at high methyl ethanoate concentration in the liquid reaction composition or due to a high concentration of inert gases (for example nitrogen and carbon dioxide) in the carbonylation reactor. The catalyst system may also have advantages of increasing rate of carbonylation when the rate of reaction is reduced by the availability of carbon monoxide in solution in the liquid reaction composition resulting from mass transfer limitations, for example due to poor agitation.

The pressure of the carbonylation reaction is suitably in the range 0.9 to 19.9 MPa, preferably 0.9 to 9.9 MPa, most preferably 1.4 to 7.5 MPa. The temperature of the carbonylation reaction is suitably in the range 100 to 300 DEG C, preferably in the range 150 to 220 DEG C.

Ethanoic acid may advantageously be used as a solvent for said carbonylation reaction.

Corrosion metals, particularly nickel, iron and chromium should preferably be kept to a minimum in the liquid reaction composition as these may have an adverse effect in said carbonylation reaction.

The carbonylation process of the present invention may be performed as a batch or continuous process, preferably as a continuous process and may be performed in any suitable reactor, known to those skilled in the art.

The methyl ethanoate product may be removed from the carbonylation reactor by withdrawing liquid reaction composition and separating the methyl ethanoate product by one or more flash and/or fractional distillation stages from the other components of the liquid reaction composition such as iridium catalyst, ruthenium and/or osmium and/or indium promoter, methyl iodide, water and unconsumed reactants (methanol, DME, carbon monoxide and intermediates, such as ethanoic acid) which may be recycled to the reactor to maintain their concentrations in the liquid reaction composition. The methyl ethanoate product may also be removed as a vapour from the stream exiting the carbonylation reactor. According to a preferred embodiment of the present invention, during the above separation stage, water is selectively removed from/or added to the carbonylation process in order to maintain the aforementioned operating conditions.

According to a preferred embodiment of the present invention, the separated methyl ethanoate from the stream exiting the carbonylation reactor is purified to remove ethanoic acid, carbonylation catalyst and water, before its introduction into the alcohol synthesis unit. After purification and before introduction into the alcohol synthesis unit, the methyl ethanoate stream contains preferably less than 0.1 ppm wt of carbonylation catalyst, more preferably less than 0.05 ppm wt, most preferably less than 0.005 ppm wt. After purification and before introduction into the alcohol synthesis unit, the methyl ethanoate stream contains preferably less than 5 wt% of ethanoic acid, more preferably less than 1 wt%, even more preferably less than 0.1wt% and most preferably less than 100ppm by weight. The methyl ethanoate feed is also preferably free from residual carboxylic acid and has a total acid number (TAN) of less than 100, more preferably less than 50 and most preferably less than 5 (where according to the present invention, TAN is the amount (mg) of Potassium Hydroxide needed to fully neutralize 1g of the sample material).

After purification and before introduction into the alcohol synthesis unit, the ethanoate stream contains preferably less than 20 wt% of water, more preferably less than 2 wt%, most preferably less than 0.2 wt%.

The methyl ethanoate fed into the alcohol synthesis unit is preferably purified of halide compounds. This purification treatment can be achieved in by any appropriate method known to those skilled in the art. For example, halides and iodides can be removed in the liquid phase using silver salts (either homogeneous or supported) on either an ionexchange resin or a zeolite.

Furthermore, the methyl ethanoate feed is preferably vapourised prior to contacting the alcohol synthesis catalyst by either, heating the methyl ethanoate in a separate vapouriser prior to having contact with the synthesis gas or, by heating the methyl ethanoate within the synthesis gas (e.g. either in a separate vessel or on a prebed to the alcohol synthesis reactor). The feed mixture including recycles entering the alcohol synthesis unit (e.g. the synthesis gas, together with the methyl ethanoate) is preferably more than 10C, preferably more than 20C above its dew point temperature. For the purposes of the present invention and appending claims, the 'dew point temperature' is defined as being a threshold temperature. For example, for a given mixture, at a given pressure, if the system temperature is raised to above the dew point temperature, the mixture will exist as a dry gas.

The applicants have unexpectedly found a preferred mode of operation whereby the methyl ethanoate/methanol close boiling point mixture from the carbonylation unit can also advantageously be used together with the methyl ethanoate as a feed to the alcohol synthesis unit; this is particularly advantageous because it considerably simplifies the purification process.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

## Claims

1. Process for the conversion of synthesis gas into ethanol **characterised by** the following steps:
1) converting synthesis gas and methyl ethanoate in an alcohol synthesis unit to produce methanol and ethanol,
2) separating the methanol from the ethanol in step 1,
3) introducing methanol from step 2 into an etherification unit to produce dimethyl ether,
4) introducing dimethyl ether, from step 3 together with carbon monoxide, into a carbonylation unit, in the presence of a iridium based carbonylation catalyst, to produce methyl ethanoate,
5) introducing methyl ethanoate, from step 4, into the alcohol synthesis unit of step 1, and
6) recovering ethanol from step 2.

2. Process for the conversion of a hydrocarbon feedstock(s) into ethanol, wherein the hydrocarbons are first converted into synthesis gas, which is subsequently converted into ethanol, **characterised by** the following steps:
1) introducing a hydrocarbon feedstock, into a synthesis gas reactor, in order to produce a mixture of carbon oxide(s) and hydrogen (synthesis gas),
2) converting synthesis gas, from step 1, together with hydrogen and methyl ethanoate in an alcohol synthesis unit to produce methanol and ethanol,
3) separating the methanol from the ethanol in step 2,
4) introducing methanol from step 3 into an etherification unit, to produce dimethyl ether,
5) introducing dimethyl ether, from step 4 together with carbon monoxide, into a carbonylation unit, in the presence of a iridium based carbonylation catalyst, to produce methyl ethanoate,
6) introducing methyl ethanoate, from step 5, into the alcohol synthesis unit of step 2, and
7) recovering ethanol from step 3.

3. Process according to any of the preceding claims wherein the molar ratio, ([H2]-[CO2])/([CO]+[CO2]), (where the concentrations of relevant components are expressed in volume percent or mole percent), of the fresh synthesis gas feed stream fed into the alcohol synthesis unit is greater than 3.8.

4. Process according to any of the preceding claims wherein the molar ratio, ([H2]-[CO2])/([CO]+[CO2]), of the fresh synthesis gas feed stream fed into the alcohol synthesis unit is less than 6.0.

5. Process according to any of the preceding claims wherein the alcohol synthesis unit is operated at a temperature of more than 180°C.

6. Process according to any of the preceding claims wherein the alcohol synthesis unit is operated at a temperature of less than 290°C.

7. Process according to any of the preceding claims wherein the alcohol synthesis unit is operated at a pressure of more than 2 MPa.

8. Process according to any of the preceding claims wherein the alcohol synthesis unit is operated at a pressure of less than 10 MPa.

9. Process according to any of the preceding claims wherein the alcohol synthesis unit comprises a methanol synthesis catalyst which is a mixture of copper, zinc oxide, and a promoter such as, chromia or alumina.

10. Process according to claim 9 wherein the alcohol synthesis unit also comprises a hydrogenation catalyst selected from
(i) a precious metal based catalyst, comprising of at least one noble metal from Group VIII of the periodic table (CAS version, for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII noble metal,
(ii) a copper-based catalyst which is different from the methanol catalyst (for example a copper chromite or a mixed copper metal oxide based catalyst wherein the second metal can be Cu, Zn, Zr or Mn), and
(iii) mixtures thereof.

11. Process according to claim 10 wherein the hydrogenation catalyst mixed with the methanol catalyst is a supported copper based catalyst which is different from the methanol catalyst and which comprises copper and promoters, such as cobalt and/or manganese and/or chromium, supported on zinc oxide.

12. Process according to any of the preceding claims wherein the stream exiting the alcohol synthesis unit comprises an ethanol/ethyl ethanoate azeotrope which is recovered and recycled to the alcohol synthesis unit.

13. Process according to any of the preceding claims wherein the etherification unit comprises an etherification catalyst which is chosen amongst heterogeneous catalysts, such as aluminium oxides and aluminium silicates, zeolites and heteropolyacids (such as silicotungstenic acid).

14. Process according to any of the preceding claims wherein the process for the production of methyl ethanoate by carbonylation of DME comprises contacting DME with carbon monoxide, in the liquid reaction composition, in a carbonylation reactor wherein, the liquid reaction composition comprises:
(a) ethanoic acid, (b) methyl ethanoate, (c) an iridium catalyst, (d) methyl iodide, (e) water and (f) at least one promoter.

15. Process according to claim 14 wherein the concentration of water in the liquid reaction composition of the carbonylation unit is in the range 0.1 to 15% by weight, more preferably 1 to 10% by weight, most preferably 1 to 6.5% by weight.

16. Process according to any of claims 14 and 15, wherein the iridium carbonylation catalyst concentration in the liquid reaction composition is in the range 100 to 6000 ppm by weight of iridium, more preferably 700 to 3000 ppm by weight of iridium.

17. Process according to any of claims 14 to 16 wherein the promoter is selected from the group consisting of ruthenium, osmium, rhenium, cadmium, mercury, zinc, gallium, indium and tungsten, and is present in the liquid reaction composition at a molar ratio of promoter : iridium of [0.5 to 15] : 1.

18. Process according to any of claims 14 to 17 wherein the concentration of methyl iodide in the liquid reaction composition is in the range 1 to 20% by weight, preferably 5 to 16% by weight.

19. Process according to any of the preceding claims wherein the methyl ethanoate stream exiting the carbonylation unit is purified to remove ethanoic acid, carbonylation catalyst and water, before its introduction into the alcohol synthesis unit.

20. Process according to claim 19 wherein the methyl ethanoate stream contains less than 0.1 ppm wt of carbonylation catalyst, preferably less than 0.05 ppm wt, more preferably less than 0.005 ppm wt.

21. Process according to any of claims 19 and 20 wherein the methyl ethanoate stream contains less than 5 wt% of ethanoic acid, preferably less than 1 wt%, more preferably less than 0.1wt% and most preferably less than 100ppm by weight.

22. Process according to any of claims 19 to 21 wherein the methyl ethanoate stream contains preferably less than 20 wt% of water, more preferably less than 2 wt%, most preferably less than 0.2 wt%.

23. Process according to any of the preceding claims wherein the methyl ethanoate stream is vapourised prior to its introduction into the alcohol synthesis unit.

24. Process according to any of the preceding claims wherein the feed mixture including recycles entering the alcohol synthesis unit is more than 10C, preferably more than 20C above its dew point temperature.
